# EUROPÄISCHE PATENTANMELDUNG

(11) **EP 2 489 408 A1**
(43) Veröffentlichungstag der Anmeldung: **22.08.2012**
(21) Anmeldenummer: 11000211.0
(22) Anmeldetag: 13.01.2011
(51) Int. Cl.: A61P 15/06, A23L 1/305, A61K 31/198, A61P 43/00

(54) **Aminosäurezusammensetzung und deren Verwendung zur Behandlung von fetaler Wachstumsrestriktion (IUGR) und zur parenteralen Ernährung von extrem Frühgeborenen**

(71) Anmelder: Tchirikov, Michael, 55116 Mainz (DE)
(72) Erfinder: Tchirikov, Michael, 55116 Mainz (DE)
(74) Vertreter: Patentanwälte Bitterich, Dr. Keller, Schwertfeger

(57) **Zusammenfassung**

Zusammensetzung enthalten sind.
7. Zusammensetzung nach einem der Ansprüche 1 bis 6, dadurch gekennzeichnet, dass die Aminosäuren in einer 10 %-Glucoselösung vermischt sind.
8. Verwendung einer Zusammensetzung nach einem der Ansprüche 1 bis 7 für die Herstellung eines Arzneimittels zur Behandlung einer fetalen Wachstumsrestriktion (IUGR).
9. Verwendung einer Zusammensetzung nach einem der Ansprüche 1 bis 7 für die Herstellung eines Arzneimittels zur parenteralen Ernährung bei extrem Frühgeborenen in der 24. bis 34. Schwangerschaftswoche.

## Beschreibung

Die vorliegende Erfindung betrifft eine Aminosäurezusammensetzung und deren Verwendung zur Behandlung von intrauteriner fetaler Wachstumsrestriktion (IUGR), und zur parenteralen Ernährung von extrem Frühgeborenen in der 24. bis 34. Schwangerschaftswoche.

Eine intrauterine Wachstumsretardierung (Restriktion) des Fötus (Intrauterine Growth Restriction (IUGR)) wird hauptsächlich durch eine Plazentainsuffizienz der Mutter hervorgerufen und stellt eine schwerwiegende Komplikation während der Schwangerschaft dar. IUGR ist eine der Hauptursachen für Frühgeburt, welche wiederum zum Tode des Frühgeborenen führen kann (Goldenberg et al., Epidemology and causes of preterm birth, Lancet 2008;371:75-84). Zu den anderen Ursachen der IUGR zählen genetische Vorbelastungen, mütterliche Erkrankungen oder starker Drogenkonsum der Mutter. Eine intrauterine Wachstumsrestriktion wird während der Schwangerschaft durch eine Ultraschallkontrolle festgestellt.

Die fetale Wachstumsretardation kommt in ungefähr in 3 bis 5 % aller Schwangerschaften vor. Dabei stellt die chronische Hypoxia in IUGR-Föten die Hauptursache für die neonatalen Komplikationen dar (Salihu et al., Is small for gestational age a marker of future fetal survival in utero? Obstet Gynecol 2006;107:851-856).

In vielen Fällen erhöht sich die Sterberate beim Krankheitsverlauf bei IUGR-Föten um das 3- bis 10-fache. Etwa 25 bis 40 % aller absterbenden intrauterinen Föten sind IUGR-Föten (Salihu et al., Is small for gestational age a marker of future fetal survival in utero? Obstet Gynecol 2006;107:851-856).

Die fetale Wachstumsretardation während der Schwangerschaft verschlechtert die Entwicklung des Neugeborenen und des Kindes beträchtlich (Pryor J. et al., Development and behaviour in adolescents born small-for-gestational-age. J Paediatr Child Health 1995;31:403-407). Kinder mit IUGR leiden an depressiven und kognitiven Krankheiten (Räikkönen et al., Depression in young adults with very low birth weight; the Helsinki study of very-low-birth-weight adults. Arch Gen Psychiatry 2008; 65:290-296). Die IUGR-Situation während der Schwangerschaft führt zu Veränderungen beim fetalen Programmablauf ("Barker-Hypothese"). Ferner wird die fetale Wachstumsretardation auch als Ursache für die Entwicklung von metabolischem Syndrom sowie für ein erhöhtes Risiko für die Entwicklung von koronaren Herzerkrankungen, Infarkten und Diabetes mellitus Typ 2 angesehen (Barker DJ et al., The fetal origins of adult hypertension, J Hypertens Suppl 1992;10:39-44; Fetal nutrition and cardiovascular disease in adult life. Lancet 1993;341:938-941; Fetal origins of coronary heart disease. BMJ 1995;311:171-174; In utero programming of chronic disease. Clin Sci 1998;95:115-128; Fetal programming of coronary heart disease, Trends Endocrinol Metab 2002;13:364-368).

Aufgrund des aktiven Transports von Aminosäuren von der Mutter zum Fötus ist die Konzentration der Aminosäuren im Nabelschnurblut des Fötus normalerweise um das 1,1- bis 3-fache höher als die Aminosäurenkonzentration des Blutplasmas der Mutter. Bei der IUGR ist der Aminosäurentransport durch die Plazenta reduziert, für einige Aminosäuren sogar ganz besonders (Ronzoni et al., The effect of a maternal infusion of amino acids on umbilical uptake in pregnancies complicated by intrauterine growth restriction. Am J Obstet Gynecol 2002:187:741-6).

Ferner wurde festgestellt, dass auch der aktive Glucosetransport durch die Mikrovilli-Membran von Synytiotrophoblasten in IUGR-Föten signifikant vermindert ist (Jansson et al., Glucose transport and system A activity in syncytiotrophoblast microvillous and basal plasma membranes in intrauterine growth restriction. Placenta 2002;23:392-399).

Nach der Diagnose von IUGR sind die Optionen für eine Behandlung nur sehr beschränkt (Van Kamp et al., Complication of intrauterine intravascular transfusion for fetal anemia due to maternal red-cell alloimmunization. Am J Obstet Gynecol 2005;192:171-177). Die Patienten mit einer IUGR werden frühzeitig per Sectio entbunden, um einen intrauterinen Fruchttod durch die Mangelversorgung infolge einer Plazentainsuffizienz zu vermeiden. Es gibt keine nachgewiesene kausale Methode für die Behandlung der Plazentainsuffizienz, da eine "insuffiziente" Plazenta in der Frühschwangerschaft mit der folgenden fetalen Wachstumsretardierung am Ende des II. Trimenoms entsteht. Eine kontinuierliche Aminosäuren- und Glukose-Infusion in der Nabelschnurvene über ein subkutan implantiertes Portsystem konnte in Studien das fetale Wachstum verbessern und die Schwangerschaft um mehrere Wochen prolongieren (M. Tchirikov et al, treatment of growth-restricted human fetuses with amino acids and glucose supplementation through a chronic fetal intravascular perinatal port system, Case Report Eur Surg Res 2010;45:45-49).

Auch stehen nur begrenzte Therapiemöglichkeiten nach der extremen Frühgeburt zur Verfügung. In Betracht kommt eine Behandlung mit einer Aminosäurenlösung, welche die Versorgung der Frühgeborenen sicherstellen soll, wobei die Zusammensetzung der Aminosäuren, die der mütterlichen Milch ähnlich ist, sich deutlich von der im fetalen Plasma in utero unterscheidet. Das hat sehr wahrscheinlich negative Folgen für den Frühgeborenen (zum Beispiel veränderte Hirnentwicklung und Myelinisierung, signifikante Reduzierung des IQs, besondere mit der IUGR in der Anamnese). Zurzeit werden diese Komplikationen nur durch die Frühgeburt erklärt, wobei eine falsche parenterale Ernährung mit Aminosäuren, die auf der Milch-Basis basiert, selten in Betracht gezogen wird.

Aminosäurezusammensetzungen als solche sind generell für unterschiedliche Anwendungsgebiete im Stand der Technik hinreichend bekannt (man vergleiche beispielsweise die DE 698 31 609 T2, DE 693 32 433 A1, DE 699 33 738 T2, EP 2 116 238 A1).

Im dem für die vorliegende Anmeldung nächstliegenden Stand der Technik wurde eine kommerziell erhältliche Aminosäurezusammensetzung (Vaminolact) zur Behandlung von IUGR-Patienten über ein perinatales Port-System eingesetzt (M. Tchirikov et al., treatment of growth-restricted human fetuses with amino acids and glucose supplementation through a chronic fetal intravascular perinatal port system, Case Report Eur Surg Res 2010;45:45-49). Insbesondere wird ein intravaskuläres Ergänzungssystem beschrieben, um Nährstoffe an einem IUGR-Fötus zur Verfügung zu stellen, um das fetale Wachstum zu verbessern und eine Verlängerung der Schwangerschaft sicherzustellen, da IUGR-Föten im Uterus nicht selbst die Fähigkeit besitzen, heranzuwachsen. Eine langfristige Ergänzung mit Nährstoffen über das perinatale Port-System durch transkutane Verabreichung von Aminosäuren und Glukose in die Nabelvene unter Verwendung des Port-Systems führte zu einer Erhöhung des fetalen Körpergewichtes und hatte das Potential zur Schwangerschaftsprolongation.

Obgleich der Einsatz des Port-Systems eine verbesserte Verabreichungsmöglichkeit zur Zufuhr der Aminosäuren an den Fötus darstellt, gilt es nach wie vor, die Aminosäurezusammensetzung zu optimieren, um die Wirkung und somit Behandlungserfolg zu verbessern.

Ferner besteht nach wie vor ein Bedarf zur extrauterinen Behandlung von extrem Frühgeborenen, d.h. solchen Kindern, die in der 24. bis 34. Schwangerschaftswoche geboren wurden. Diese haben in vielen Fällen wenig Überlebenschance und müssen unter erschwerten Bedingungen behandelt werden. Im Falle einer extremen Frühgeburt wird beim Frühgeborenen angestrebt, eine in utero-ähnliche Umgebung durch die Verwendung von Inkubatoren herzustellen. Es stehen zurzeit keine geeigneten Aminosäurenlösungen mit Aminosäurenkonzentration zur Verfügung, die einer fetalen Aminosäurenkonzentration in utero ähnlich sind. Dies ist unbefriedigend.

Vor diesem Hintergrund ist es Aufgabe der vorliegenden Erfindung, die Bereitstellung von Aminosäuren durch eine gegenüber bekannten Zusammensetzungen optimierte Aminosäurelösung zu verbessern, um dadurch eine intrauterine Wachstumsretardation des Fötus oder die Probleme bei extrem früh Geborenen und die damit einhergehenden Folgen zu vermindern oder zu verhindern. Eine optimierte in utero-ähnliche Aminosäurenmischung soll ferner bei extremen Frühgeborenen die parenterale Ernährung verbessern und Komplikationen der Frühgeburt verhindern oder vermindern.

Diese Aufgabe wird durch eine Aminosäurezusammensetzung gemäß Anspruch 1 gelöst.

Die vorliegende Erfindung betrifft eine Aminosäurezusammensetzung zur Behandlung einer fetalen Wachstumsrestriktion (IUGR) und zur parenteralen Ernährung von extrem Frühgeborenen in der 24. bis 34. Schwangerschaftswoche, umfassend eine Lösung mit mindestens 15 Aminosäuren, ausgewählt aus einer Gruppe bestehend aus folgenden Aminosäuren oder deren Aminosäurenanalogen:
Isoleucin, Leucin, Lysin, Methionin, Cystein, Phenylalanin, Threonin, Tryptophan, Valin, Arginin, Histidin, Ornithin, Glycin, Alanin, Prolin, Aspartat, Asparagin, Glutamat, Glutamin, Serin, Tyrosin, Taurin, Hydroxyprolin, Citrullin, wobei die in der Lösung enthaltenen Aminosäuren in Konzentrationen oder Konzentrationsverhältnissen vorliegen, die Bereichen liegen, die den physiologischen Aminosäurenkonzentrationen oder Konzentrationsverhältnissen im Blutplasma des Fötus in utero entsprechen.

Umfasst sind auch Substitute dieser Aminosäuren wie z.B. chemisch modifizierte oder künstliche Aminosäuren, Analoge, Homologe oder Derivate davon. Vorzugsweise liegen die Aminosäuren als Gemisch in Lösung vor.

Je nach Anwendungsfall und/oder Zusammensetzung können einzelne oder mehrere Aminosäuren in hochdosierter Form vorliegen und werden bei Anwendung mit Wasser oder einer Glukoselösung auf die gewünschte Verabreichungskonzentration verdünnt.

Die Wahl der einzelnen Aminosäuren in der erfindungsgemäßen Lösung und deren Konzentrationsbereiche bzw. Konzentrationsverhältnisse kann von unterschiedlichen Faktoren abhängen. Zum einen hängt es davon ab, in welcher Schwangerschaftswoche sich der Fötus befindet oder wann die extreme Frühgeburt stattgefunden hat. Zum anderen spielen auch praktische Erwägungen eine Rolle, beispielsweise die Stabilität oder künstliche Synthetisierbarkeit einzelner Aminosäuren. Gegebenenfalls kann es notwendig sein, Aminosäuren wie Glutamin, Hydroxyprolin oder Citrullin durch künstliche Analoge oder andere Aminosäuren zu ersetzen, oder aus der Lösung ganz heraus zu lassen.

Je nach Wahl der einzelnen in der Mischung vorliegenden Aminosäuren kann es notwendig sein, die Aminosäurenkonzentrationen in der Lösung anzupassen. Ziel ist es jedoch, den physiologischen Konzentrationen oder Konzentrationsverhältnissen des Nabelschnurbluts des Fötus möglichst nahe zukommen. Eine oder mehrere natürlich vorkommende Aminosäuren können durch eine künstliche oder modifizierte Aminosäure ersetzt werden. Demnach umfasst der hier verwendete Begriff "Analog" sowohl künstliche als auch modifizierte Aminosäuren oder Substitute.

Die Verabreichungsvolumina und somit die erwünschten Endkonzentrationen der Aminosäurenzusammensetzung werden gegebenenfalls durch Verdünnung eingestellt.

Die Aminosäurenkonzentration im Blutplasma des Fötus in utero kann über gängige Methoden, beispielsweise durch direkte Entnahme von der Nabelschnurermittelt werden. Dem Fachmann stehen hierbei Untersuchungsmethoden zur Verfügung, welche für den Fötus wenig risikobehaftet sind. Aus dem Blut des Fötus wird das Blutplasma isoliert und die darin enthaltenen Aminosäuren analysiert. Noch unproblematischer ist die Bestimmung der Aminosäurenkonzentration im Blutplasma der Mutter. Hier kann eine einfache venöse Abnahme und anschließende Bestimmung erfolgen.

Im Prinzip ist es möglich, für jedes Mutter/Fötus-Paar die ideale Aminosäurenkonzentration experimentell zu ermitteln. Es hat sich jedoch herausgestellt, dass innerhalb der in dieser Anmeldung angegebenen Konzentrationsbereiche der Aminosäuren die erwünschten Erfolge erzielt werden.

Interessanterweise weist der intrauterine Fötus in seinem Nabelschnurblut eine höhere Aminosäurenkonzentration auf als die entsprechenden Aminosäuren im maternalen Blutplasma. Auf Grundlage der physiologischen Aminosäurenkonzentration des Fötus wurden die erfindungsgemäße Aminosäurezusammensetzung entwickelt und die Konzentrationen entsprechend angepasst. Der Behandlungserfolg kann auf diese Weise bei IUGR signifikant gesteigert, eine Wachstumsretardation könnte vermindert, die Schwangerschaft verlängert werden. Die Verwendung einer physiologischen Zusammensetzung mit einer Auswahl der oben aufgeführten Aminosäuren und einem Energieträger wie Glukose soll die Komplikationen einer IUGR oder die Symptome extrem Frühgeborener verhindern oder zumindest vermindern.

Gleiches trifft auch zu für die parenterale Ernährung von extrem frühgeborenen aber der 23. Schwangerschaftswoche, bevorzugt in der 24. bis 34. Schwangerschaftswoche, bevorzugt in der 24. bis 30. SSW, besonders bevorzugt in der 24. bis 28. SSW.

In der Tabelle 1 unten sind die Konzentrationsverhältnisse der einzelnen Aminosäuren beim Fötus, der Mutter sowie einer bevorzugten Ausführungsform der erfindungsgemäßen Zusammensetzung zusammengefasst. Ferner sind die Aminosäurenkonzentrationsverhältnisse der Aminosäuren im Nabelschnurblut des Fötus im Vergleich zum Blutplasma der Mutter gezeigt.

**Tabelle 1**

| Bezeichnung | Fötus mg/l | Mutter mg/l | Fötus µmol/l | Mutter µ mol/l | Verhältnis Fötus/Mutter | **Erfindung** g/l | **Erfindung Gew.-%** |
|---|---|---|---|---|---|---|---|
| Isoleucin | 9,07 | 6,00 | 69,11 | 45,72 | 1.51 | **4,53** | **1,93** |
| Leucin | 16,35 | 11,28 | 124,64 | 86,00 | 1,45 | **8,17** | **3,48** |
| Lysin | 53,15 | 22,13 | 363,59 | 151,39 | 2,40 | **26,58** | **10,73** |
| Methionin | 4,93 | 2,69 | 33,04 | 18,05 | 1,83 | **2,46** | **0,99** |
| Cystein | 5,00 | | 33,0 | | | | **0,99** |
| Phenylalanin | 12,20 | 8,32 | 73,83 | 50,35 | 1,47 | **6,10** | **2,33** |
| Threonin | 36,43 | 21,00 | 305,85 | 176,33 | 1,73 | **18,22** | **8,14** |
| Tryptophan | 11,72 | 6,74 | 57,37 | 33,00 | 1,74 | **5,86** | **2,05** |
| Valin | 27,70 | 18,20 | 236,48 | 155,37 | 1,52 | **13,85** | **6,24** |
| Arginin | 15,35 | 8,45 | 88,11 | 48,49 | 1,82 | **7,67** | **2,86** |
| Histidin | 17,98 | 14,07 | 115,89 | 90,66 | 1,28 | **8,99** | **3,53** |
| Ornithin | 13,35 | 5,88 | 100,97 | 44,46 | 2,27 | **6,67** | **2,83** |
| Glycin | 18,48 | 11,68 | 246,23 | 155,56 | 1,58 | **9,24** | **5,39** |
| Alanin | 31,88 | 24,06 | 357,79 | 270,03 | 1,33 | **15,94** | **8,36** |
| Prolin | 20,06 | 13,29 | 174,25 | 115,41 | 1,51 | **10,03** | **4,56** |
| Aspartat | 5,73 | 2,56 | 43,04 | 19,25 | 2,24 | **2,86** | **1,21** |
| Asparagin | 10,52 | 3,50 | 79,07 | 26,28 | 3,01 | **5,26** | **2,22** |
| Glutamat | 23,61 | 19,77 | 160,44 | 134,38 | 1,19 | **11,80** | **4,75** |
| Glutamin | 77,01 | 64,63 | 526,90 | 442,21 | 1,19 | **38,50** | **15,54** |
| Serin | 15,94 | 11,13 | 151,70 | 105,87 | 1,43 | **7,97** | **3,81** |
| Tyrosin | 12,97 | 7,36 | 71,56 | 40,64 | 1,76 | **6,48** | **2,38** |
| Taurin | 19,74 | 5,91 | 157,73 | 47,26 | 3,34 | **9,87** | **4,30** |
| Hydroxyprolin | 4,10 | 2,29 | 31,29 | 17,50 | 1,79 | **2,05** | **0,87** |
| Citrullin | 2,80 | 2,80 | 15,96 | 16,00 | 1,00 | **1,40** | **0,52** |

Beachtenswert ist, dass bei dem Aminosäurenkonzentrationsverhältnis zwischen dem Fötus und der Mutter insbesondere die Aminosäuren Asparagin und Taurin signifikant, d.h. um mehr als das 3-fache erhöht sind. Ferner sind auch die Aminosäuren Lysin, Ornithin und Aspartat um mehr als das 2-fache beim Fötus gegenüber der Mutter signifikant erhöht. In einer bevorzugten Ausführungsform ist die erfindungsgemäße Zusammensetzung deshalb dadurch gekennzeichnet, dass die Aminosäurenkonzentration für Asparagin und/oder Taurin dem Verhältnis der Aminosäurenkonzentration im Nabelschnurblut des Fötus zu der Aminosäurenkonzentration im Blutplasma der Mutter für die Aminosäuren Asparagin und/oder Taurin mit wenigstens 1 : 2, vorzugsweise wenigstens 1 : 3 entspricht.

Entsprechend den physiologischen Verhältnissen beim Fötus kann die erfindungsgemäße Zusammensetzung auch anhand der Gewichtsmassen angegeben werden. In der folgenden Tabelle 2 sind die bevorzugten und besonders bevorzugten Konzentrationsbereiche für die einzelnen Aminosäuren für die erfindungsgemäße Aminosäurezusammensetzung angegeben, wobei die Gesamtmenge der Aminosäuren 100 Gew.-% beträgt:

**Tabelle 2**

| Aminosäure | Bevorzugter Konzentrationsbereich | | Besonders bevorzugter Konzentrationsbereich | |
|---|---|---|---|---|
| Isoleucin | 1,35 - 7,00 | Gew.-% | 1,50 - 3,50 | Gew.-% |
| Leucin | 2,43 - 8,00 | Gew.-% | 2,50-5,00 | Gew.-% |
| Lysin | 7,51 -15,32 | Gew.-% | 8,00 - 12,00 | Gew.-% |
| Methionin | 0,69 -1,42 | Gew.-% | 0,75 - 1,20 | Gew.-% |
| Cystein | 0,1-3,00 | Gew.-% | 0,5 - 1,00 | Gew.-% |
| Phenylalanin | 1,63 - 3,33 | Gew.-% | 1,75 - 2,75 | Gew.-% |
| Threonin | 3,00 -11,62 | Gew.-% | 6,00-10,00 | Gew.-% |
| Tryptophan | 1,00 - 2,93 | Gew.-% | 1,50 - 2,50 | Gew.-% |
| Valin | 4,37 - 8,91 | Gew.-% | 5,50 - 7,00 | Gew.-% |
| Arginin | 2,01 - 9,00 | Gew.-% | 2,75 - 3,25 | Gew.-% |
| Histidin | 2,47 - 5,04 | Gew.-% | 3,00 - 4,00 | Gew.-% |
| Ornithin | 1,98 - 5,50 | Gew.-% | 2,55 - 3,50 | Gew.-% |
| Glycin | 3,77 - 7,69 | Gew.-% | 3,77 - 7,69 | Gew.-% |
| Alanin | 5,86 - 11,95 | Gew.-% | 4,50 - 6,50 | Gew.-% |
| Prolin | 3,19 - 8,00 | Gew.-% | 4,00-5,50 | Gew.-% |
| Aspartat | 0,85 - 3,00 | Gew.-% | 2,50 - 2,95 | Gew.-% |
| Asparagin | 1,56 - 5,00 | Gew.-% | 1,56 - 5,00 | Gew.-% |
| Glutamat | 3,32 - 8,50 | Gew.-% | 3,50 - 5,00 | Gew.-% |
| Glutamin | 1,00 - 20,00 | Gew.-% | 10,50 - 18,00 | Gew.-% |
| Serin | 2,66 - 5,44 | Gew.-% | 3,00 - 4,50 | Gew.-% |
| Tyrosin | 1,00 - 3,40 | Gew.-% | 1,75 - 3,00 | Gew.-% |
| Taurin | 0,26-6,14 | Gew.-% | 3,50 - 5,50 | Gew.-% |
| Hydroxyprolin | 0,10 -1,25 | Gew.-% | 0,50 - 1,00 | Gew.-% |
| Citrullin | 0,10 - 0,74 | Gew.-% | 0,50 - 0,55 | Gew.-% |

In einer bevorzugten Ausführungsform der erfindungsgemäßen Zusammensetzung sind Taurin und/oder Asparagin mit einer Konzentration von wenigstens 5 g/l in der Zusammensetzung enthalten.

In einer bevorzugten Ausführungsform sind die oben erwähnten Aminosäuren durch Analoge oder durch modifizierte Aminosäuren ersetzt. Bestimmte Aminosäuren wie z.B. Glutamin sind in Lösungen recht instabil, so dass diese in peptidischer Form verwendet werden müssen, beispielsweise als Dipeptide oder Polypeptide. Daher sind von der Erfindung als solche Aminosäuren umfasst, welche die oben erwähnten "natürlichen" Aminosäuren ersetzen oder ersetzen können. Diese sind im Stand der Technik hinreichend bekannt.

In einer weiteren bevorzugten Ausführungsform enthält die erfindungsgemäße Zusammensetzung neben den zuvor genannten Aminosäuren oder Aminosäureanalogen zusätzlich Mikroelemente und/oder Spurenelemente. Bevorzugte Mikroelemente oder Spurenelemente sind: Chrom (Cr),Cobalt (Co), Eisen (Fe), Iod (I), Kupfer (Cu), Mangan (Mn), Molybdän (Mo), Selen (Se), Zink (Zn), Fluor (F9, Silicium (Si), Arsen (As), Nickel (Ni), Zinn (Sn), Vanadium (V).

In einer weiteren Ausführungsform wird die erfindungsgemäße Aminosäurezusammensetzung in einer Zuckerlösung, vorzugsweise einer 10 %-Glucoselösung vermischt.

Je nach Anwendungsgebiet kann es sinnvoll sein, die Stoffmengen der eingesetzten Aminosäuren in entsprechenden Volumina zu lösen und dem Fötus/Kind zu applizieren, um eine unnötige Volumenbelastung des Herzens zu vermeiden.

In dem nachfolgenden Beispiel ist eine bevorzugte Ausführungsform der erfindungsgemäßen Zusammensetzung dargestellt.

### Beispiel 1

Ein von IUGR (fetaler Wachstumsretardation) betroffener Fötus wird mit einer erfindungsgemäßen Zusammensetzung über ein intravaskuläres perinatales Port-System versorgt (M. Tchirikov et al., Treatment of growth-restricted human fetuses with amino acids and glucose supplementation through a chronic fetal intravascular preinatal port system, Case Report Eur Surg Res 2010;45:45-49). Die Zusammensetzung enthielt die folgenden Aminosäuren in den angegebenen Konzentrationen:

| | |
|---|---|
| Isoleucin | 4,53 g/l |
| Leucin | 8,17 g/l |
| Lysin | 26,58 g/l |
| Methionin | 2,46 g/l |
| Phenylalanin | 6,10 g/l |
| Threonin | 18,22 g/l |
| Tryptophan | 5,86 g/l |
| Valin | 13,85 g/l |
| Arginin | 7,67 g/l |
| Histidin | 8,99 g/l |
| Ornithin | 6,67 g/l |
| Glycin | 9,24 g/l |
| Alanin | 15,94 g/l |
| Prolin | 10,03 g/l |
| Aspartat | 2,86 g/l |
| Asparagin | 5,26 g/l |
| Glutamat | 11,80 g/l |
| Glutamin | 38,50 g/l |
| Serin | 7,97 g/l |
| Tyrosin | 6,48 g/l |
| Taurin | 9,87 g/l |
| Hydroxyprolin | 2,05 g/l |
| Citrullin | 1,40 g/l |

zusammen mit einer 10 %-Glucoselösung. Das Port-System ermöglicht eine permanente Versorgung des IUGR-Fötus mit Aminosäuren über die Nabelschnur.

Die Behandlung des Fötus erfolgte über tägliche Infusionen mit einer Aminosäurelösung (5 ml/h; 25 ml/Tag) plus einer 10 %-Glucoselösung (25 ml/Tag).

### Beispiel 2

Ein in der 25. Schwangerschaftswoche extrem früh geborenes Kind wird mit einer erfindungsgemäßen Zusammensetzung intravenös behandelt. Die Zusammensetzung enthält die folgenden Aminosäuren in den angegebenen Konzentrationen:

| | |
|---|---|
| Isoleucin | 1,93 Gew.-% |
| Leucin | 3,48 Gew.-% |
| Lysin | 10,73 Gew.-% |
| Methionin | 0,99 Gew.-% |
| Cystein | 0,99 Gew.-% |
| Phenylalanin | 0,99 Gew.-% |
| Threonin | 2,33 Gew.-% |
| Tryptophan | 8,14 Gew.-% |
| Valin | 2,05 Gew.-% |
| Arginin | 6,24 Gew.-% |
| Histidin | 2,86 Gew.-% |
| Ornithin | 3,53 Gew.-% |
| Glycin | 2,83 Gew.-% |
| Alanin | 5,39 Gew.-% |
| Prolin | 8,36 Gew.-% |
| Aspartat | 4,56 Gew.-% |
| Asparagin | 1,21 Gew.-% |
| Glutamat | 2,22 Gew.-% |
| Glutamin | 4,75 Gew.-% |
| Serin | 15,54 Gew.-% |
| Tyrosin | 3,81 Gew.-% |
| Taurin | 2,38 Gew.-% |
| Hydroxyprolin | 0,87 Gew.-% |
| Citrullin | 0,52 Gew.-% |

## Patentansprüche

1. Aminosäurezusammensetzung zur Behandlung einer fetalen Wachstumsrestriktion (IUGR) und zur parenteralen Ernährung von extrem Frühgeborenen in der 24. bis 34. Schwangerschaftswoche, umfassend eine Lösung mit mindestens 15 Aminosäuren, ausgewählt aus einer Gruppe bestehend aus folgenden Aminosäuren oder deren Aminosäurenanalogen:
Isoleucin, Leucin, Lysin, Methionin, Phenylalanin, Threonin, Tryptophan, Valin, Arginin, Histidin, Ornithin, Glycin, Alanin, Prolin, Aspartat, Asparagin, Glutamat, Glutamin, Serin, Tyrosin, Taurin, Hydroxyprolin, Citrullin,
**dadurch gekennzeichnet, dass** die in der Lösung enthaltenen Aminosäuren in Konzentrationen oder Konzentrationsverhältnissen vorliegen, die Bereichen liegen, die den physiologischen Aminosäurenkonzentrationen oder Konzentrationsverhältnissen im Blutplasma des Fötus in utero entsprechen.

2. Zusammensetzung nach Anspruch 1, **dadurch gekennzeichnet, dass** die Aminosäurenkonzentration für Asparagin und/oder Taurin dem Verhältnis der Aminosäurenkonzentration des Fötus zu der Aminosäurenkonzentration im Plasma der Mutter für die Aminosäuren Asparagin und/oder Taurin mit wenigstens 1 : 2, vorzugsweise wenigstens 1 : 3 entspricht.

3. Zusammensetzung nach Anspruch 1 oder 2, **dadurch gekennzeichnet, dass** die ausgewählten Aminosäuren innerhalb folgender Konzentrationsbereiche in der Zusammensetzung vorhanden sind, wobei die Gesamtmenge der Aminosäuren 100 Gew.% beträgt:
| | | |
|---|---|---|
| Isoleucin | 1,35 - 7,00 | Gew.-% |
| Leucin | 2,43 - 8,00 | Gew.-% |
| Lysin | 7,51 - 15,32 | Gew.-% |
| Methionin | 0,69 - 1,42 | Gew.-% |
| Cystein | 0,1 - 3,00 | Gew.-% |
| Phenylalanin | 1,63 - 3,33 | Gew.-% |
| Threonin | 3,00-11,62 | Gew.-% |
| Tryptophan | 1,00 - 2,93 | Gew.-% |
| Valin | 4,37 - 8,91 | Gew.-% |
| Arginin | 2,01 - 9,00 | Gew.-% |
| Histidin | 2,47 - 5,04 | Gew.-% |
| Ornithin | 1,98 - 5,50 | Gew.-% |
| Glycin | 3,77 - 7,69 | Gew.-% |
| Alanin | 5,86 - 11,95 | Gew.-% |
| Prolin | 3,19 - 8,00 | Gew.-% |
| Aspartat | 0,85 - 3,00 | Gew.-% |
| Asparagin | 1,56 - 5,00 | Gew.-% |
| Glutamat | 3,32 - 8,50 | Gew.-% |
| Glutamin | 1,00 - 20,0 | Gew.-% |
| Serin | 2,66 - 5,44 | Gew.-% |
| Tyrosin | 1,00 - 3,40 | Gew.-% |
| Taurin | 0,26 - 6,14 | Gew.-% |
| Hydroxyprolin | 0,10 - 1,25 | Gew.-% |
| Citrullin | 0,10 - 0,74 | Gew.-% |

4. Zusammensetzung nach Anspruch 1 bis 3, **dadurch gekennzeichnet, dass** die ausgewählten Aminosäuren innerhalbfolgender Konzentrationsbereiche in der Zusammensetzung vorhanden sind, wobei die Gesamtmenge der Aminosäuren 100 Gew.-% beträgt:
| | | |
|---|---|---|
| Isoleucin | 1,50 - 3,50 | Gew.-% |
| Leucin | 2,50 - 5,00 | Gew.-% |
| Lysin | 8,00 - 12,00 | Gew.-% |
| Methionin | 0,75 - 1,20 | Gew.-% |
| Cystein | 0,5 - 1,00 | Gew.-% |
| Phenylalanin | 1,75 - 2,75 | Gew.-% |
| Threonin | 6,00 - 10,00 | Gew.-% |
| Tryptophan | 1,50 - 2,50 | Gew.-% |
| Valin | 5,50 - 7,00 | Gew.-% |
| Arginin | 2,75 - 3,25 | Gew.-% |
| Histidin | 3,00 - 4,00 | Gew.-% |
| Ornithin | 2,55 - 3,50 | Gew.-% |
| Glycin | 3,77 - 7,69 | Gew.-% |
| Alanin | 4,50 - 6,50 | Gew.-% |
| Prolin | 4,00 - 5,50 | Gew.-% |
| Aspartat | 2,50 - 2,95 | Gew.-% |
| Asparagin | 1,56 - 5,00 | Gew.-% |
| Glutamat | 3,50 - 5,00 | Gew.-% |
| Glutamin | 10,50 - 18,00 | Gew.-% |
| Serin | 3,00 - 4,50 | Gew.-% |
| Tyrosin | 1,75 - 3,00 | Gew.-% |
| Taurin | 3,50 - 5,50 | Gew.-% |
| Hydroxyprolin | 0,50 - 1,00 | Gew.-% |
| Citrullin | 0,50 - 0,55 | Gew.-% |

5. Zusammensetzung nach einem der Ansprüche 1 bis , **dadurch gekennzeichnet, dass** die Aminosäuren in folgenden Konzentrationen in folgenden Konzentrationen in der Zusammensetzung vorhanden sind:
| | | |
|---|---|---|
| Isoleucin | 1,93 | Gew.-% |
| Leucin | 3,48 | Gew.-% |
| Lysin | 10,73 | Gew.-% |
| Methionin | 0,99 | Gew.-% |
| Cystein | 0,99 | Gew.-% |
| Phenylalanin | 0,99 | Gew.-% |
| Threonin | 2,33 | Gew.-% |
| Tryptophan | 8,14 | Gew.-% |
| Valin | 2,05 | Gew.-% |
| Arginin | 6,24 | Gew.-% |
| Histidin | 2,86 | Gew.-% |
| Ornithin | 3,53 | Gew.-% |
| Glycin | 2,83 | Gew.-% |
| Alanin | 5,39 | Gew.-% |
| Prolin | 8,36 | Gew.-% |
| Aspartat | 4,56 | Gew.-% |
| Asparagin | 1,21 | Gew.-% |
| Glutamat | 2,22 | Gew.-% |
| Glutamin | 4,75 | Gew.-% |
| Serin | 15,54 | Gew.-% |
| Tyrosin | 3,81 | Gew.-% |
| Taurin | 2,38 | Gew.-% |
| Hydroxyprolin | 0,87 | Gew.-% |
| Citrullin | 0,52 | Gew.-% |

6. Zusammensetzung nach einem der Ansprüche1 bis 5, **dadurch gekennzeichnet, dass** Taurin und/oder Asparagin mit einer Konzentration von wenigstens 5 g/l in der Zusammensetzung enthalten sind.

7. Zusammensetzung nach einem der Ansprüche 1 bis 6, **dadurch gekennzeichnet, dass** die Aminosäuren in einer 10 %-Glucoselösung vermischt sind.

8. Verwendung einer Zusammensetzung nach einem der Ansprüche 1 bis 7 für die Herstellung eines Arzneimittels zur Behandlung einer fetalen Wachstumsrestriktion (IUGR).

9. Verwendung einer Zusammensetzung nach einem der Ansprüche 1 bis 7 für die Herstellung eines Arzneimittels zur parenteralen Ernährung bei extrem Frühgeborenen in der 24. bis 34. Schwangerschaftswoche.
